Europäisches Patentamt

European Patent Office

Office européen des brevets

(19)

(11) Publication number: **0 171 871**

**A1**

(12)

# EUROPEAN PATENT APPLICATION

(21) Application number: **85303045.0**

(22) Date of filing: **30.04.85**

(51) Int. Cl.⁴: **G 01 N 33/78**
**G 01 N 33/577, G 01 N 33/543**

(30) Priority: **29.05.84 US 614586**

(43) Date of publication of application:
**19.02.86 . Bulletin 86/8**

(84) Designated Contracting States:
**DE FR GB IT NL**

(71) Applicant: **WARNER-LAMBERT COMPANY**
**201 Tabor Road**
**Morris Plains New Jersey 07950(US)**

(72) Inventor: **Kelly, Kenneth A.**
**1908 Delta Drive**
**Arlington Texas 76012(US)**

(72) Inventor: **Barnes, Wayne R.**
**18788 Linden Avenue**
**Grayside Illinois 60030(US)**

(72) Inventor: **Guzzaldo, Carole**
**121 Simmons**
**Coppell Texas 75019(US)**

(72) Inventor: **Morrison, Julie D.**
**14100 Montfort Drive Apt. 2146**
**Dallas Texas 75240(US)**

(74) Representative: **Douma, Anno Do et al,**
**Organon International B.V. Postbus 20**
**NL-5340 BH Oss(NL)**

(54) A method for determining the unsaturated binding capacity of a serum protein.

(57) There is disclosed a method of assaying the degree of unsaturation of thyroid hormone binding capacity of a serum protein, which comprises:

(a) mixing a solution of serum protein with a labelled tri-iodothyronine (T3) tracer in the presence of a secondary T3 binder which is attached to a solid support;

(b) admixing the resulting solution while rotating the solution for a sufficient time to cause a selective complexing to the secondary binder of the T3 tracer which is not bound to the serum protecin; and

(c) separating the T3 tracer bound to the solid support from the solution and determining the amount of T3 tracer which is either (1) the T3 tracer bound to the serum protein in solution or (2) the T3 tracer bound to the solid support.

## A METHOD FOR DETERMINING THE UNSATURATED BINDING CAPACITY OF A SERUM PROTEIN

The present invention relates to a method for determining the unsaturated binding capacity of certain serum proteins and particularly thyroxine-binding globulin.

T3 uptake tests are designed to assess the unsaturated binding capacity of certain serum proteins, primarily thyroxine-binding globulin (TBG) as well as, thyroxine binding prealbumin (TBPA) and albumin. TBG has a greater affinity for thyroxine (T4) than tri-iodothyronine (T3). The degree of unsaturation of TBG in a sample is assessed by the addition of a T3 tracer which reacts with free TBG sites without displacing endogenous T4 already bound to TBG. The greater the degree of unsaturation the more T3 tracer is bound by sample with reduced uptake of tracer to a secondary binder. For example, in hyperthyroidism where the patient's TBG is relatively saturated, elevated T3 tracer uptake to the secondary binder is recorded. Conversely, when the degree of unsaturation is high as in low T4 levels in hypothyrodism, and in samples with elevated TBG levels such as during pregnancy, reduced T3-tracer uptake to the secondary binder is observed.

Various diagnostic tests are known in the art for determining thyroid function. These tests include the basal metabolism test, the thyroid uptake test and various colorimetric and chemical procedures for determining the level of thyroxine iodine in the blood. Among the most accurate tests available are the diagnostic tests which utilize radioisotope labeled hormone to indirectly determine the level of thyroid hormones, thyroxine ($C_{15}H_{11}I_4NO_4$), and tri-iodothyronine ($C_{15}H_{11}I_3NO_4$) present in body fluids. Specifically, these tests include a test commonly referred to as the T3 test which measures the unsaturated binding capacity of thyrobinding globulin and other proteins within a body fluid such as blood, and the test commonly referred to as the T4 test which measures the total quantity of hormone within a sample of blood serum.

Both of these tests include the steps of reacting a mixture of the radioisotope labeled hormone with a solution containing the patients sample of hormone and thyrobinding globulin in the presence of a secondary binding agent, separating the resulting thyrobinding globulin containing bound hormone from the resulting unbound hormone, and counting the radioactivity of either the bound or unbound hormone fraction. This counting procedure will indirectly indicate the amount of endogenous hormone which is bound to the natural globulin and protein binding sites within the blood.

Major variations in T3 uptake methodology relate to the selection of the secondary binding agents. These include ion exchange resins, sephadex, activated charcoal and silicate which bind free T3 by physical adsorption. See Witherspoon et al, The Triiodothyronine Uptake Test: An Assessment of Methods. Clinical Chemistry Vol.27, p.1272-1276, 1981. A primary disadvantage of the currently available separation methods described is that binding of the hormones to the separation materials is both time and temperature dependent, so that the binding of the hormones to these materials increases with time and increasing temperatures.

For example, in the case of ion exchange resins, as time increases, much of the protein-bound thyroid hormone is ultimately bound to the resin. In addition, from one to two hours are required for complete binding to take place with the ion exchange resins, further limiting the number of measurements which are possible in the laboratory.

In contrast, when using physical adsorption techniques that employ charcoal on the solid phase, such as beads coated with charcoal, the procedure requires careful timing and controlled temperatures to enable systematic and slow adsorption of the T3 tracer onto the coated charcoal. To accelerate the reaction rate the beads may be optionally rotated.

In an effort to enhance selective adsorption of the T3 tracer and reduce interference from the presence of lipids in serum samples one technique has been developed using antibodies specific to T3 bound to the solid support. While this method provides improved specificity it requires long reaction times to obtain coupling of the T3 tracer to the natural binding sites within the antibody. Exemplary times vary from 30 to 60 minutes or more.

Figure I depicts the classification of various serum samples.

The present invention constitutes an improved diagnostic test for T3 uptake which measures unsaturated binding capacity of the thyroid binding globulin and other proteins within body fluids. More particularly the invention involves the use of a labeled T3 which is reacted with serum sample in the presence of a secondary T3 binder attached to a solid support, which reaction takes place while the contents are subjected to constant rotation.

More particularly, the invention involves a method for assaying the degree of unsaturation of thyroid hormone binding capacity of serum proteins, comprising mixing a solution of serum proteins with a labeled T3 tracer in the presence of a secondary T3 binder attached to a solid support, thoroughly admixing the resulting solution while rotating the solution for a sufficient time to cause a selective complexing to said secondary binder of the T3 tracer which is not bound to the serum proteins and separating the T3 tracer bound to the solid support from the solution and determining the amount of (1) the T3 tracer bound to the serum proteins in solution or (2) the T3 tracer bound to the solid support.

In accordance with the present invention, the thyroid hormone binding capacity is determined by mixing a T3 tracer with an unknown serum sample in the presence of a secondary T3 binder attached to a solid support.

The T3 tracer may be any one of a wide variety of tracers in which T3 or an appropriate analog thereof includes a "label" or "tag," such as a radioactive isotope, enzyme, fluoroescent or chemiluminescent tag, and so forth.

When the T3 is labeled with an enzyme as described, for example, by Wilheimus in U.S. Patent 3,791,932, the amount of labeled T3 can be determined by measuring enzymatic activity of the tube. When the T3 is labeled with a fluorescent material the amount of labeled T3 can be determined by measuring fluorescense as described by U.S. Patent 3,789,116 to Kay. However the labeling is accomplished, the labeled material serves as a tracer.

A particularly preferred method involves labeling the T3 with a radioactive isotope in a conventional manner. Suitable isotopes include $I^{125}$, $I^{131}$, $C^{14}$ or $H^3$. A particularly suitable isotope is the radioactive isotope of iodine, $I^{125}$, since labeling with this isotope is simple and many hospital laboratories have the equipment necessary to measure this material.

The binder or receptor which is bound to the solid support may be any one of a wide variety of thyroid hormone binders, including antibodies, serum proteins and the like. The preferred binder is an antibody which may be either polyclonal or monoclonal. Coupling to the solid support may be accomplished by standard techniques, such as active coupling and passive adsorption. One particularly preferred method involves coupling the binder to the solid support using an aldehyde compound.

The aldehyde compounds that may be used in the invention may be selected from a wide variety of monofunctional and bifunctional reagents which are water-soluble. Exemplary water-soluble monofunctional material may be represented by the formula CHO-R wherein R is hydrogen, an alkyl group, and an aryl group. Representative compounds include formaldehyde, acetaldehyde, propionaldehyde, n-butyraldehyde and benzaldehyde, and mixtures thereof, with formaldehyde being preferred.

Exemplary water-soluble bifunctional material may be presented by the formula $OHC-(R)n-CHO$, wherein R may be $-(CH_2)-$, and wherein n is an integer from 2 to 10; such as an alkyl group, for example, glutaraldehyde; monocyclic alkyl group, for example, cyclopentyl or cyclohexyl; monocyclic aryl group, for example, phenyl; bicyclic aryl group, for example, naphthyl; substituted monocyclic alkane group, or substituted monocyclic aryl group, for example, tolyl and mixtures thereof. The preferred bifunctional aldehyde is glutaraldehyde. It should be recognized that other coupling agents may be employed according to the invention.

Suitable solid support surfaces are formed from water-insoluble polymeric material capable of adsorbing the secondary T3 binder. Exemplary materials include hydrocarbon polymers such as polystyrene, polyethylene, polypropylene and polybutylene. Other suitable organic polymers include silastic rubber, polyesters, polyamides, cellulose and cellulosic derivatives, acrylates, methacrylates, and vinyl polymers such as polyvinyl chloride, and polyvinyl fluoride. Copolymers such as graft copolymers of polystyrene are also useful.

A particularly effective form for the solid support surface is a test tube, bead, fin, well or microtitre plate.

Once the reactants are placed in contact with one another, it is essential that the solution is rotated for a sufficient time for the reaction to be effectively completed. The manner in which the rotation is performed is a critical feature of this invention in order to achieve a rapid and complete reaction. Rotation is preferably performed continuously at a rate of about 150 to 300 rpm for 10 to 30 minutes and most preferably from 170 to 210 rpm for 15 to 25 minutes. The reactants are incubated at suitable temperatures to complete the reaction within the optimum time and rotation, for example from about 10 to 45°C and preferably 20 to 30°C.

It should be recognized that the extent of reaction is directly dependent upon rate of rotation, time and temperature wherein there is a reverse relationship between any two of the variables when the third variable is constant. It has been unexpectedly found that enhanced and accelerated efficiency of the reaction between the secondary binder and free T3 tracer also leads to improved competition between the secondary binder and serum proteins for T3 tracer, yielding a better resolution or distinction among different degrees of TBG unsaturation of serum samples. This result is unexpected since one would expect a consistent increase in T3 uptake values for both hypo and hyperthyroid samples without a corresponding expansion of the difference between their T3 uptake values.

Once the reaction is terminated, reaction fluid is removed such as by decantation or aspiration and the amount of T3 tracer associated with the solid support or fluid is measured to determine percent T3 uptake.

The percent binding of the T3 tracer to the immobilized binder is inversely related to the serum protein binding capacity, and the binding capacity of a known serum sample is qualitatively determined by comparing the percentage of T3 tracer bound in the unknown sample with the percentage of T3 tracer bound in a reference sample. In order to minimize interassay variations, the assay is effected with both an unknown sample and a reference sample, with a normal pooled sample being assigned a percent uptake.

The percent uptake of the unknown sample (T3 uptake) is determined as follows:

$$T3\ uptake = \frac{percent\ binding\ T3\ tracer\ in\ unknown}{percent\ binding\ T3\ tracer\ in\ reference} \times assigned\ percent\ uptake$$

The thus determined T3 uptake is a qualitative measurement of the serum binding capacity of the unknown, with such binding capacity showing a qualitative increase when the T3 uptake is below the assigned percent uptake, and a qualitative decrease when the T3 uptake is above the assigned percent uptake.

The present invention is further illustrated by the following examples. All parts and percentages in the examples as well as in the specification and claims are by weight unless, otherwise specified.

8

This example demonstrates the enhanced uptake of free T3 tracer and improved resolution of unsaturated TBG T3-binding capacity when the reaction was carried out under rotation compared to the conventional stationary mode.

Assay Procedure:

Volumes of 10μl (micro) of a hypothyroid (L40), normal (LR27.1) and hyperthyroid (H65) serum sample were pipetted into polypropylene tubes to which were coupled an anti-T3 antiserum. $^{125}I-T3$ tracer (400 1) was then added to each tube. After initial vortexing, the tubes were then either rotated at 190rpm for 20 minutes or allowed to stand for increasing incubation times. At the completion of the incubation (24°C), the contents of the tubes were decanted and radioactivity bound to the antibody-coated tubes measured. Results:

Table 1 lists the values of %T3 uptake to the antibody-coated tubes. Less than 50% of the uptake observed under rotation for 20 minutes is obtained when the reaction is carried out in a stationary mode for the same time. Although the binding increases by extending the time of incubation in a stationary mode, at 60 minutes both the absolute T3 uptake values and the spread between hyperthyroid and hypothyroid samples, (H65-L40) are much less than obtained by rotating the reaction tube for 20 minutes.

- 9 -

0171871

## TABLE 1

### Comparison of T3 Uptake Values (%)
### for reaction in a rotation versus stationary mode

| SERUM SAMPLE | ROTATION TIME 20mins @ 190rpm | STATIONARY REACTION TIME (mins) | | |
|---|---|---|---|---|
| | | 20 | 40 | 60 |
| L40 | 41.4 | 20.7 | 28.7 | 31.4 |
| LR27.1 | 49.6 | 24.3 | 31.6 | 35.8 |
| Hb5 | 59.3 | 28.2 | 35.4 | 41.3 |
| $\Delta$(H65-L40) | 17.9 | 7.5 | 6.7 | 9.9 |

## EXAMPLE II

This example demonstrates the direct dependence of % T3 uptake to the antibody-coated tubes on the speed of rotation and time of reaction.

Assay Procedure:

Volumes of 10 µl of serum samples were dispensed into anti-T3 antibody-coated polypropylene tubes, followed by 400 µl of $^{125}I\text{-}T_3$ tracer. The reaction tubes were then rotated at room temperature at 200 to 300 rpm for 10 to 30 minutes. The tubes were then decanted and tracer bound to the tubes measured.

Results:

As is shown in Table 2, increasing the speed of rotation from 200 to 300 rpm for a fixed reaction time increases the % T3 uptake to the antibody-coated tubes. Similarly, at any constant speed of rotation extending the time of reaction also linearly increases the binding of T3 tracer to the tube. However, it has been generally observed that the resolution of degree of unsaturation of TBG between any two serum samples, e.g. H65 (hyperthyroid) and L40 (hypothyroid), remains unchanged if either only the speed of rotation or the time of reaction is altered. Thus, at 250 rpm, although the % T3 uptake increases with increasing time of reaction from 10 to 30 minutes, the differences in % T3 uptake between H65 and L40 do not vary significantly. This is the desired result since the difference of % T3 uptake is an indication of differences in the degree of unsaturation of TBG between samples. Rotating the reaction mixture, while accelerating the binding of free T3 tracer to the insolubilized antibody, at the same time promotes a more efficient competition between the antibody sites (secondary binder) and unsaturated serum TBG (primary binder) for the $^{125}I\text{-}T_3$ reagent. This efficient

EXAMPLE II (cont'd)

competitive state between the two T3 binders exists in T3 uptake tests which dispense the secondary binder such as resin or silicate throughout the reaction mixture.

In particular, reaction conditions of rotation at $190 \pm 10$ rpm and time of incubation of $20 \pm 0.5$ minutes were chosen because they yielded absolute % T3 uptake values and resolution (T3U) for patient samples which correlated well with established commercial T3 uptake tests which employ physical absorption of the T3 tracer to the secondary binder.

12

0171871

TABLE 2

Effect of speed of rotation and reaction time

on the measurement of T3 uptake

| SPEED OF ROTATION (rpm) | REACTION TIME (minutes) | %T3 UPTAKE | | RESOLUTION (%T3U) |
|---|---|---|---|---|
| | | L40 | H65 | Δ(H65-L40) |
| 200 | 10 | 20.1 | 31.8 | 11.7 |
| | 20 | 29.0 | 45.3 | 16.3 |
| | 30 | 37.1 | 52.4 | 15.7 |
| 250 | 10 | 22.1 | 37.4 | 15.3 |
| | 20 | 32.8 | 49.8 | 17.0 |
| | 30 | 41.6 | 58.2 | 16.6 |
| 300 | 10 | 27.8 | 42.9 | 15.1 |
| | 20 | 37.4 | 58.2 | 20.8 |
| | 30 | 46.7 | 66.1 | 19.4 |

## EXAMPLE III

This example demonstrates the improved performance of an antibody-coated tube T3 uptake assay when carried out in a rotation mode in comparison with a similar assay in a stationary mode.

Assay Procedure:

Sixty-five patient samples, varying in degree of TBG unsaturation from pregnancy (samples 1-6), to hypothyroid (samples 7-24), to normal euthyroid (samples 25-47), to hyperthyroid (samples 48-65), as classified by NML Tri-Tab T3 Uptake test, were assayed for %T3 uptake using (1) a standard test employing a silicate adsorbent, (2) the inventive process of Example 1 (antibody-coated tube in a rotation phase) and (3) a standard test using an antibody-coated tube in a stationary phase.

The samples were assayed according to procedures specified by the manufacturers. In the NML Tri-Tab T3U test, a silicate tablet was dispersed in the mixture of sample and T3 tracer to physically adsorb free tracer not bound to serum TBG. In the Clinical Assays Gamma-Coat T3U test, the mixture of sample and T3 tracer was allowed to stand for 60 minutes prior to measurement of free T3 tracer bound to the antibody-coated tube. In the inventive T3U test, the mixture of sample and T3 tracer was rotated for 20 minutes at 190 rpm before measuring tracer bound to the tube. All assays were performed at room temperature at the same time.

Results:

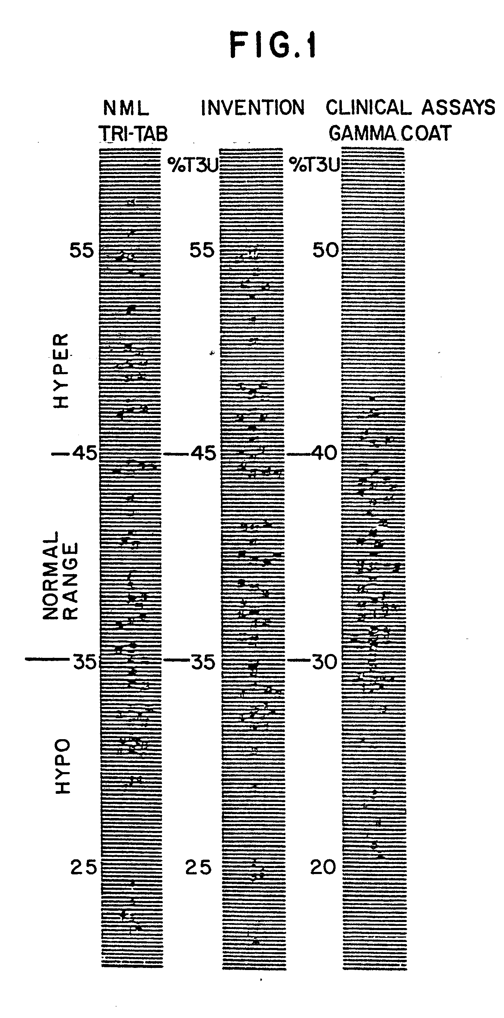

Corrected T3 uptake values were determined and plotted as shown in Figure 1. Samples are identified by number 1-65, respectively, and plotted according to their % T3 Uptake values. The normal ranges for each assay have also been indicated, so that the classification of each sample into hypothyroid, normal and hyperthyroid can be compared.

The inventive antibody coated tube assay with rotation for 20 minutes demonstrates far superior resolution with a spread of 33% between the lowest and highest % T3U measured, compared to the stationary antibody-coated tube assay (Clinical Assays) with a spread of 20 to 43% T3U. The spread in the rotation inventive test compares favorably with the NML Tri-Tab silicate test.

The spread or resolution between degrees of TBG unsaturation among patient samples is very good over the full range of T3U values when the reaction is carried out under rotation. In contrast, when the reaction is carried out in a stationary mode, even for 60 minutes, there is poor resolution in the low T3U pregnancy end of the scale and even worse distinction among hyperthyroid (high T3U) samples.

The invention being thus described, it will be obvious that the same may be varied in many ways. Such variations are not to be regarded as a departure from the spirit and scope of the invention, and all such modifications are intended to be included within the scope of the following claims.

CLAIMS:

1. A method of assaying the degree of unsaturation of thyroid hormone binding capacity of a serum protein, which comprises:

(a) mixing a solution of serum protein with a labelled tri-iodothyronine (T3) tracer in the presence of a secondary T3 binder which is attached to a solid support;

(b) admixing the resulting solution while rotating the solution for a sufficient time to cause a selective complexing to the secondary binder of the T3 tracer which is not bound to the serum protein; and

(c) separating the T3 tracer bound to the solid support from the solution and determining the amount of T3 tracer which is either (1) the T3 tracer bound to the serum protein in solution or (2) the T3 tracer bound to the solid support.

2. A method according to Claim 1, wherein the solution is rotated in step (b) at 150 to 300 rpm for 10 to 30 minutes.

3. A method according to Claim 1 or 2, wherein the solution is rotated in step (b) at 170 to 210 rpm for 15 to 25 minutes.

4. A method according to any preceding claim, wherein the serum protein is thyroid hormone binding globulin.

5. A method according to any preceding claim, wherein the serum protein is present in blood.

6. A method according to any one of Claims 1 to 4, wherein the serum protein is present in serum or plasma.

7. A method according to any preceding claim, wherein the tracer is a radioisotope label, enzyme label, fluorescent label or chemiluminescent label.

0171871

8.    A method according to any preceding claim, wherein the secondary T3 binder is a polyclonal antibody to T3.

9.    A method according to any one of Claims 1 to 7, wherein the secondary T3 binder is a monoclonal antibody to T3.

10.    A method according to any preceding claim, wherein the secondary T3 binder is an antibody that is actively bound to the solid support.

11.    A method according to any preceding claim, wherein the solid support is in the form of a test tube.

0171871

# FIG.1

NML INVENTION CLINICAL ASSAYS
TRI-TAB GAMMA COAT

%T3U    %T3U

55    55    50

HYPER

—45    —45    —40

NORMAL RANGE

—35    —35    —30

HYPO

25    25    20

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 355 293 (SMITH KLINE INSTRUMENTS LTD.) * Whole document * | 1-11 | G 01 N 33/78 G 01 N 33/577 G 01 N 33/543 |
| Y | US-A-3 654 090 (ORGANON INC.) * Column 4, lines 34-40; column 6, lines 36-40 * | 1-11 | |
| A | EP-A-0 007 744 (BECTON DICKINSON AND CO.) * Whole document * | 1,4-8, 10 | |
| A,P | WO-A-8 500 663 (K. TUNG et al.) * Whole document * | 2,3,5-10 | |
| Y | FR-A-2 514 510 (MOCHIDA PHARMACEUTICAL CO. LTD.) * Page 3, line 3 - page 4, line 3; claims 1-3 * | 1-3 | TECHNICAL FIELDS SEARCHED (Int. Cl.4) G 01 N |

The present search report has been drawn up for all claims

| Place of search THE HAGUE | Date of completion of the search 17-09-1985 | Examiner HITCHEN C.E. |
|---|---|---|